(19) Europäisches Patentamt
European Patent Office
Office européen des brevets

(11) Veröffentlichungsnummer: **0 094 665 B2**

(12) **NEUE EUROPÄISCHE PATENTSCHRIFT**

(45) Veröffentlichungstag der neuen Patentschrift: **13.03.91 Patentblatt 91/11**

(51) Int. Cl.[5]: **C07D 251/28**, C07D 251/44, C07D 251/56, C07D 251/70, C09B 62/085, B01J 19/26

(21) Anmeldenummer: **83104811.1**

(22) Anmeldetag: **16.05.83**

# (54) Verfahren und Anlage zur Herstellung einer Suspension von Cyanurchlorid in Wasser oder zur Umsetzung von Cyanurchlorid mit Ammoniak oder Aminen.

Die Akte enthält technische Angaben, die nach dem Eingang der Anmeldung eingereicht wurden und die nicht in dieser Patentschrift enthalten sind.

(30) Priorität: **18.05.82 CH 3082/82**
**24.05.82 CH 3184/82**

(43) Veröffentlichungstag der Anmeldung: **23.11.83 Patentblatt 83/47**

(45) Bekanntmachung des Hinweises auf die Patenterteilung: **01.04.87 Patentblatt 87/14**

(45) Bekanntmachung des Hinweises auf die Entscheidung über den Einspruch: **13.03.91 Patentblatt 91/11**

(84) Benannte Vertragsstaaten: **CH DE FR GB IT LI**

(56) Entgegenhaltungen:
**DE-A- 1 670 731**
**DE-A- 2 551 164**
**DE-A- 2 850 271**
**DE-A- 2 850 338**
**DE-A- 2 942 364**
**FR-A- 2 441 620**
**FR-A- 2 441 624**
**US-A- 2 779 763**
**E.Siegel "Chemistry of Synthetic Dyes", Vol VI, Tabelle 1,1972,**
**Chemical Abstracts, Vol.62, No.12, 1965,14703**

(73) Patentinhaber: **CIBA-GEIGY AG**
**Klybeckstrasse 141**
**CH-4002 Basel (CH)**

(72) Erfinder: **Punzar, Marianne**
**Gustackerstrasse 13/2**
**CH-4103 Bottmingen (CH)**
Erfinder: **Marti, Franz, Dr.**
**Hügelweg 47**
**CH-4143 Dornach (CH)**
Erfinder: **Mercier, Robert**
**Matthäusstrasse 9**
**CH-4057 Basel (CH)**
Erfinder: **Tobler, Paul**
**Gstaltenrainweg 84**
**CH-4125 Riehen (CH)**
Erfinder: **Büttiker, Rudolf, Dr.**
**Arnikastrasse 9**
**CH-4125 Riehen (CH)**

(74) Vertreter: **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun. Bräuhausstrasse 4**
**W-8000 München 2 (DE)**

EP 0 094 665 B2

## Beschreibung

Die Erfindung betrifft ein Verfahren und eine Anlage zur Herstellung einer Suspension von Cyanurchlorid in Wasser oder zur Umsetzung von Cyanurchlorid mit Ammoniak oder Aminen gemäss Oberbegriff von Patentanspruch 1 bzw. 5.

Verfahren zur Herstellung einer Suspension von Cyanurchlorid in Wasser sind schon seit längerem bekannt. So beschreibt die DE-B-1 670 731 ein Verfahren, bei dem man geschmolzenes Cyanurchlorid in Wasser einlaufen lässt. Dieses Verfahren wird z.B. durchgeführt, indem man an der Innenwand eines senkrecht stehenden Zylinders einen Wasserfluss erzeugt und das geschmolzene Cyanurchlorid am Kopf des Zylinders über eine zum Versprühen geeignete heizbare Düse einbringt. Das Verfahren ist jedoch noch mit einigen Nachteilen behaftet. So treten z.B. Probleme dadurch auf, dass sich auf der Wasseroberfläche leicht eine Schicht von festem Cyanurchlorid bildet, und es gelingt mit diesem Verfahren nicht, eine Cyanurchloridsuspension mit einer durchschnittlichen Korngrösse von weniger als 20 µm herzustellen. Ausserdem sublimiert in der genannten Vorrichtung ein Teil des Cyanurchlorids, was leicht zur Verstopfung der Düse führen kann. In der DE-B-2 850 242 ist ein Verfahren zur Herstellung von Suspensionen von Cyanurchlorid in Wasser beschrieben, in dem man flüssiges Cyanurchlorid durch eine Sprühdüse, die sich in dem Kopf eines rohrförmigen Behälters befindet, in diesen Behälter einsprüht, wobei dieser rohrförmige Behälter oben geschlossen oder verschliessbar ist und sich nach unten brustförmig zu einer Ausflussöffnung verjüngt und bei dem das Wasser durch eine oder mehrere Düsen, die sich oberhalb der Verjüngung befinden, austritt und eine Flüssigkeitsschicht längs der gesamten Kammerwände bis zur Düse für das Cyanurchlorid bildet. In der DE-A-2850271 ist der für dieses Verfahren geeignete Rohrreaktor näher beschrieben.

Auch dieses Verfahren vermag noch nicht vollends zu befriedigen. da die in der Praxis oft gewünschte kleine Korngrösse des Cyanurchlorids in der erhaltenen Suspension nicht erreicht werden kann. Ausserdem können nach diesem Verfahren keine feinteiligen Suspension höherer Konzentration. z.B. mit mehr als 20 Gew.% Cyanurchlorid, hergestellt werden, da bei höheren Konzentrationen Verkrustungen auftreten. Schliesslich erfordert dieses Verfahren ein spezielles Reaktionsgefäss.

In der DE-B-2 850 331 ist auch bereits ein Verfahren zur Umsetzung von geschmolzenem Cyanurchlorid mit Aminen beschrieben. Auch für dieses Verfahren ist jedoch das vorstehend beschriebene spezielle Reaktionsgefäss erforderlich.

Der vorliegenden Erfindung lag die Aufgabe zugrunde, ein Verfahren zur Verfügung zu stellen, welches die erwähnten Nachteile der bekannten Verfahren möglichst weitgehend vermeidet. Insbesondere sollte das neue Verfahren es ermöglichen, eine Suspension von Cyanurchlorid in Wasser mit einer durchschnittlichen Korngrösse unter 20 µm und einer Konzentration von mehr als 20 Gew.% Cyanurchlorid herzustellen.

Diese Aufgabe wird durch das erfindungsgemässe Verfahren gelöst. Es gestattet die Herstellung von z.B. 30%igen Suspensionen von Cyanurchlorid in Wasser mit einer Korngrösse zwischen 0 und 20 µm. Die Anlage umfarst eine besonders geeignete Sprühvorrichtung, welche jedoch in den üblichen Reaktionsgefässen zum Einsatz kommen kann. Schliesslich ist es mit dem erfingungsgemässen Verfahren möglich, auf einfache Art und Weise Cyanurchlorid direkt mit wässrigen Lösungen oder Suspensionen von Ammoniak oder Amminen umzusetzen. Gegenstand der vorliegenden Erfindung ist ein Verfahren zur Herstellung einer Suspension von Cyanurchlorid in Wasser oder zur Umsetzung von Cyanurchlorid mit Ammoniak oder Aminen, indem man geschmolzenes Cyanurchlorid mit einer Temperatur zwischen 146 und 190°C in Wasser oder eine wässrige Ammoniak- oder Aminlösung oder -suspension mit einer Temperatur zwischen 0 und 25°C einsprüht, dadurch gekennzeichnet, dass man das geschmolzene Cyanurchlorid durch eine Düse, welche in die Flüssigkeit eintaucht, in Wasser oder eine wässrige Ammoniak- oder Aminlösung oder -suspension sprüht, wobei beim Starten des Einsprühvorgangs der Flüssigkeitspegel im Umgebungsbereich der Düse soweit abgesenkt wird bis die Düsenspitze nicht mehr in die Flüssigkeit eintaucht, und dass der Flüssigkeitspegel unmittelbar nach dem Starten wieder angehoben wird.

Vorzugsweise liegt die Temperatur der Cyanurchloridschmelze zwischen 155 und 180°C.

Als Amine kommen für das erfindungsgemässe Verfahren aliphatische, aromatische oder heterocyclische Amine in Frage, und zwar primäre und sekundäre Amine, wie z.B. Methylamin, Dimethylamin, Ethylamin, Diethylamin, Propylamin, Isopropylamin, n-Butylamin, Isobutylamin, tert.-Butylamin, 3-Methoxypropylamin, α-Aminoisobutyronitril, Anilin, n-Ethylanilin, 2-, 3- oder 4-Aminopyridin sowie insbesondere Farbstoffe oder optische Aufheller sowie deren Zwischenprodukte, welche primäre oder sekundäre Aminogruppen aufweisen. Es handelt sich beispielsweise um Naphthylaminsulfosäuren, Aminonaphtholsulfosäuren, Aminoanthrachinone, Aminostilbensulfonsäuren oder um Azo- oder Anthrachinonfarbstoffe mit mindestens. einer unsubstituierten oder monosubstituierten Aminogruppe.

Die Amine und das Cyanurchlorid werden im allgemeinen in etwa stöchiometrischen Mengen eingesetzt, doch ist auch ein geringer Ueberschuss, z.B. bis zu 10% an einer der Komponenten möglich.

Das Gewichtsverhältnis von Ammoniak oder Aminen zu Wasser kann in weitem Bereich schwanken. Im allgemeinen werden 5- bis 50%ige, vorzugsweise 10- bis 40%ige Lösungen oder Suspensionen eingesetzt.

Falls gewünscht oder er :orderlich kann der wässrigen Suspension der Amine ein üblicher Dispergator zugesetzt werden, um die Feinverteilung des Amins zu verbessern.

Das Versprühen des geschmolzenen Cyanurchlorids erfolgt vorzugsweise durch eine geheizte Düse. Es empfiehlt sich, die Bedingungen beim Versprühen so zu wählen, dass die durchschnittliche Teilchengrösse des Cyanurchlorids nach dem Versprühen insbesondere zwischen 5 und 20 µm liegt.

Das erfindungsgemässe Verfahren kann diskontinuierlich oder vorzugsweise kontinuierlich durchgeführt werden.

Mit dem erfindungsgemässen Verfahren lassen sich feinkömige Suspensionen mit mehr als 50 Gew.-% Cyanurchlorid herstellen. Je nach Versuchsbedingungen können die Primärteilchen sich zwar wieder zu losen Agglomeraten zusammenballen, jedoch zerfallen diese schon bei geringer mechanischer Beanspruchung wieder. Langwierige Mahloperationen wie bei den eingangs beschriebenen Verfahren des Standes der Technik sind nicht erforderlich. Man erhält Suspensionen mit einer Korngrösse von 5 bis 20 µm, welche praktisch frei von Hydrolyseprodukten sind.

Sprüht man das Cyanurchlorid statt in Wasser in wässrige Lösungen oder Suspensionen von Ammoniak oder Aminen, so erhält man direkt die Reaktionsprodukte des Cyanurchlörids mit 1 oder 2 Mol Amin, je nach den eingesetzten Mengenverhältnissen.

Das erfindungsgemässe Verfahren wird vorzugsweise in der im Patentanspruch 5 beschriebenen erfindungsgemässen Anlage durchgeführt. Im folgenden wird ein Ausführungsbeispiel einer solchen erfindungsgemässen Anlage anhand der Zeichnung näher erläutert. Es zeigen :

Fig. 1 eine blockschematische Übersichtsdarstellung der Anlage,

Fig. 2 eine Sprühanordnung in geschnittener Detaildarstellung,

Fig. 3 einen Längsschnitt durch die eigentliche Sprühdüse der Fig. 2 und

Fig. 4 einen Querschnitt durch die Sprühdüse nach der Linie A-A der Fig. 3.

Die Anlage besteht darstellungsgemäss aus zwei Kreisläufen, und zwar aus einem Schmelzekreislauf SMK und einem Suspensionskreislauf SUK. Der Schmelzekreislauf umfasst einen Schmelzkessel 1 für das Cyanurchlorid, ein Filter 2, eine Dosier- (und Umwälz-)Pumpe 3 und ein Druckhalteventil 4 sowie diverse nicht näher bezeichnete Leitungen zur Verbindung der genannten Teile. Der Suspensionskreislauf SUK umfasst einen Kristallisator (Wassertank) 5, eine Mühle 6, eine Umwälzpumpe 7 und einen Kühler 8 sowie ebenfalls nicht bezeichnete Verbindungsleitungen. Ferner sind eine Zufuhrleitung 9 zur Zufuhr von Flüssigphase (Wasser oder wässrige Ammoniak- oder Aminlösung oder -suspension) in den Kristallisator 5 sowie eine Leitung 10 zur Suspen sionsentnahme aus dem Suspensionskreislauf SUK vorgesehen. Schliesslich umfasst die Anlage noch als zentrales Element eine Sprühanordnung 11. die an den Schmelzekreislauf SMK angeschlossen ist und zum Einsprühen von Schmelze (Cyanurchlorid) in die im Kristallisator befindliche flüssige Phase dient.

Der Aufbau der Sprühanordnung 11 geht aus den Fig. 2 und 3 hervor. Sie umfasst eine nach unten offene, etwa flaschenförmige Tauchglocke 21 und eine in dieser koaxial angeordnete Sprühdüse 22, welche mittels einer Uberwurfmutter 23 und einer Gewindehülse 24 lösbar in der Tauchglocke befestigt ist. Die Tauchglocke 21 weist etwa in der Hälfte ihrer Höhe zwei gegenüberliegende Wasserzufuhranschlüsse 25 auf. Etwas darüber befinden sich zwei Anschlüsse 26 und 27 zur Zufuhr und Abfuhr von Druckgas (Stickstoff). Ferner ist noch ein Anschluss 28 für einen Druckfühler vorgesehen. Die Stickstoffzu- und Abfuhr ist über zwei Ventile 46 und 47 gesteuert.

Die eigentliche Sprühdüse 22 besteht im wesentlichen aus drei koaxialen Zylindern 31, 32 und 33, die zwischen sich zwei Ringräume 34 und 35 bilden. Der innerste Zylinder 33 bildet die eigentliche Düse. Er weist an seinem oberen Ende einen Anschluss 36 zur Zufuhr von Schmelze und einen Anschluss 37 für einen Temperaturfühler auf, in sein unteres Ende ist ein Düsenkörper 38 eingeschraubt.

Der mittlere Zylinder 32 bildet einen Heizmantel für die Düse. Er ist mit zwei gegenüberliegenden Anschlüssen 39 und 40 für ein Heizmedium – gewöhnlich Ol – versehen. Der zwischen ihm und dem innersten Zylinder 33 befindliche Ringraum 35 ist durch eine nicht ganz bis unten reichende Trennwand 41 in zwei kommunizierende Hälften geteilt, so dass das Heizmedium den genannten Heizmantel ausreichend gleichmässig durchströmt.

Der äusserste Zylinder 31 bildet einen Wärmeisoliermantel für die Düse, welcher eine Aufwärmung der Flüssigkeit, in welche die Sprühanordnung im Betrieb eintaucht, verhindert.

Der äussere Ringraum 34 ist dazu über einen Anschluss 42 an eine Vakuum-Quelle (z.B. Vakuum-Netz oder dgl.) angeschlossen.

Die gesamte Sprühanordnung 11 ist an ihren diversen Zuleitungen aufgehängt und so relativ zum Kristallisator 5 befestigt, dass die Sprühdüse 22 unterhalb des Flüssigkeitsniveaus 43 zu liegen kommt, also in die Flüssigkeit eintaucht.

Der Betrieb der beschriebenen Anlage ist wie folgt.

Das geschmolzene Cyanurchlorid wird über den Filter 2 im Schmelzekreislauf SMK gepumpt. Ein Teilstrom. der mit Hilfe des Druckhalteventils 4 und der Dosierpumpe 3 geregelt wird, gelangt in die Sprühanordnung 11 und wird in Wasser, bzw. in wässriger Ammoniak- oder Aminlösung oder -suspension im Kristallisator 5 zerstäubt. Der Inhalt des Kristallisators wird über den Suspensionskreislauf SUK zirkuliert und dabei gekühlt.

Die Mühle 6 sorgt für die Zerkleinerung von allfälligen Agglomeraten. Nachdem die Suspension den geforderten Feststoffgehalt erreicht hat, bzw. die Umsetzung von Cyanurchlorid mit Ammoniak oder Aminen beendet ist, wird ein Teilstrom via Leitung 10 kontinuierlich abgezogen und durch die entsprechende Menge Wasser bzw. wässrige Ammoniak- oder Aminlösung oder -suspension via Leitung 9 ersetzt.

Zum Starten des Sprühvorgangs wird das Stickstoffzufuhrventil 46 geöffnet und dadurch ein Stickstoffpolster in der Tauchglocke 21 erzeugt, welches den Flüssigkeitsspiegel innerhalb der Tauchglocke so weit nach unten drückt, bis die Spitze der Sprühdüse 22 nicht mehr eintaucht. In diesem Zustand werden die Schmelzezuleitungen innerhalb der Sprühdüse und die Düsenspitze (der Düsenkörper 38) aufgeheizt. Nunmehr wird die Cyanurchloridschmelze in die Sprühdüse eingespeist und gleichzeitig durch Offnen des Entlüftungsventils 47 das Stickstoffpolster abgebaut und damit das Niveau in der Tauchglocke angehoben, so dass die Sprühdüse dann in die Flüssigkeit eintaucht. Auf diese Weise kann ein Sublimieren der Schmelze sowie das Entweichen von Hydrolyseprodukt (Salzsäure) verhindert werden.

Durch die Niveausteuerung in der Tauchglocke braucht die Sprühdüse nicht beweglich angeordnet zu sein, was in vielerlei Hinsicht vorteilhaft ist. Gleichzeitig gewährleistet dies und die Vakuum-Isolation der Sprühdüse, dass nicht unerwünscht viel Wärme an die umgebende Flüssigkeit abgegeben wird. Beides stellt bei der Herstellung von Cyanurchlorid-Suspensionen eine Voraussetzung dar, da die notwendige Heiztemperatur relativ hoch (160-190°) ist und die Neigung zur Hydrolyse mit steigender Wasser-Temperatur rasch zunimmt.

Mittels der Wasserzufuhranschlüsse 25 kann frisches Wasser bzw. Ammoniak- oder Aminlösung in die Tauchglocke 21 eingespeist werden, so dass der unmittelbare Umgebungsbereich der Sprühdüse 22 noch zusätzlich gekühlt werden kann.

Die Steuerung des Flüssigkeitsniveaus in der Tauchglocke kann durch entsprechendes Offnen und Schliessen der Ventile 46 und 47 von Hand oder auch mittels einer nicht dargestellten auto matischen Steuerung erfolgen.

Die folgenden Beispiele dienen der weiteren Erläuterung der Erfindung. Teile bedeuten Gewichtsteile und die Temperaturen sind in Grad Celsius angegeben.

Beispiel 1

36 Teile geschmolzenes Cyanurchlorid mit einer Temperatur von 160° werden durch eine Druckdüse in eine Dispergierkolonne, welche mit 108 Teilen 2-[(4-Amino-2-ureidophenyl)-azo]-naphthalin-3,6,8-trisulfosäure als 17%ige wässrige Lösung gespeist wird, so eingesprüht, dass sich die Druckdüse unterhalb des Flüssigkeitsspiegels befindet. Die Reaktionslösung erwärmt sich dabei von Zimmertemperatur auf ca. 30 bis 40°. Sie wird in einen Kessel gepumpt, wo unter Konstanthaltung des pH-Wertes von 4 bis 4,5 mit Natronlauge die Reaktion zu Ende geführt wird. 10 Minuten nach beendeter Cyanurchloriddosierung wird die entstandene Dichlortriazinverbindung mit Ammoniak zum Reaktivfarbstoff der Formel

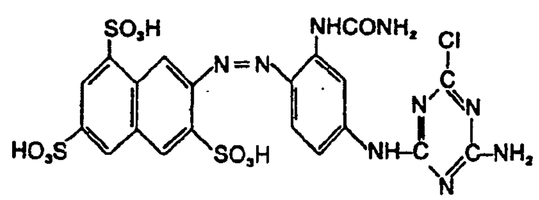

umgesetzt. Man erhält den Farbstoff in gleicher Reinheit, wie wenn er auf übliche Weise hergestellt wurde.

Beispiel 2

35 Teile Cyanurchloridschmelze von 160° werden durch eine Druckdüse in eine mit 60 Teilen 1-Amino-8-naphthol-3,6-disulfosäure als 30%ige wässrige Anschlämmung von 0° gespeiste Dispergierkolonne unter Niveau eingesprüht. Die Reaktionsmasse erwärmt sich dabei auf ca. 10°. Sie wird in einen Kessel gepumpt, wo bei 5 bis 10° unter Konstanthaltung des pH-Wertes von 1,5 bis 2,0 mit Natronlauge die Reaktion zu Ende geführt wird. Eine Stunde nach beendeter Dosierung ist kein freies Amin mehr nachweisbar. Eine in üblicher

Weise titrimetrisch mit Diazoniumsalzlösung gekuppelte Probe ergibt eine Ausbeute von über 95% d.Th. Die so hergestellte Dichlortriazinylamino-naphtholsulfosäure kann direkt zur Herstellung von Reaktivfarbstoffen verwendet werden.

Beispiel 3

60 Teile Cyanurchloridschmelze von 160° werden durch eine Druckdüse unter Niveau in eine Dispergierkolonne eingesprüht. Simultan dosiert man stöchiometrische Mengen ($\frac{1}{2}$ Mol pro Mol Cyanurchlorid) einer 10%igen wässrigen Lösung des Natriumsalzes von 4,4'-Diaminostilben-2,2'-disulfosäure mit einer Temperatur von 0° in die genannte Kolonne.

Die Reaktionsmasse wird kontinuierlich in einen Kessel gepumpt, wo bei einer mit Eis auf unter 20° gehaltenen Temperatur mit Natronlauge der pH konstant bei 4.5 gehalten wird. Die so hergestellte Verbindung N,N'-Bis-(4,6-dichlor-1,3,5-triazin-2-yl)-4,4'-diaminostilben-2,2'-disulfosäure-di-Na-Salz kann direkt zu optischen Aufhellern weiterverarbeitet werden und gibt übliche Ausbeuten und Qualitäten.

Beispiel 4

60 Teile Cyanurchloridschmelze werden wie in Beispiel 3 mit einer 30%igen wässrigen Dispersion von 4,4'-Diaminostilben-2,2'-disulfosäure-di-Na-Salz umgesetzt. Die daraus hergestellten optischen Aufheller erhält man mit nahezu quantitativer Ausbeute.

Beispiel 5

45 Teile Cyanurchloridschmelze von 160° werden analog Beispiel 3 mit 72 Teilen einer 20%igen wässrigen Isopropylamin-Lösung bei 0 bis 5° umgesetzt. Im Reaktionskessel wird mit ca. 33 Teilen 30%iger Natronlauge unter Konstanthaltung des pH die entstehende Salzsäure neutralisiert. Nach einer Stunde, wenn praktisch keine Natronlauge mehr verbraucht wird, kann analytisch (Nitrittiter) vollständige Umsetzung festgestellt werden.

Das so hergestellte Dichlor-isopropylaminotriazin wird in üblicher Weise mit Aminen, z.B. mit Athylamin, zu Herbiziden umgesetzt.

Beispiel 6

45 Teile Trichlortriazin-Schmelze (160°) werden durch eine Druckdüse unter Niveau in eine mit 21 Teilen einer 30%igen wässrigen Ammoniaklösung gespiesene Dispergierkolonne eingesprüht. Das Reaktionsgemisch wird kontinuierlich in einen Kessel gepumpt, wo bei ca. 25° unter Konstanthalten eines pH-Wertes von 8,5 bis 9,0 mit Natronlauge die Substitution zu Ende geführt wird. Das in beinahe quantitativen Mengen erhaltene Amino-dichlortriazin kann direkt zur Herstellung eines Reaktivfarbstoffes verwendet werden.

Beispiel 7

60 Teile Trichlortriazin-Schmelze (160°) werden durch eine Druckdüse in eine mit 134 Teilen 1.3-Phenylendiamin-4-sulfosäure als 30%ige wässrige Anschlämmung von 0° gespiesene Dispergierkolonne unter Niveau eingesprüht. Die Reaktionsmasse erwärmt sich dabei auf ca. 10°. Sie wird kontinuierlich in einen Kessel gepumpt, wo bei 10° unter Konstanthaltung des pH-Wertes von 5,0 mit Natronlauge die Reaktion zu Ende geführt wird. Das für Reaktivfarbstoffe weiterverwendete 2,4-Bis-(3-amino-4-sulfoanilino)-6-chlor-1,3,5-triazin entsteht in einer Ausbeute von mehr als 85%.

Beispiel 8

60 Teile Trichlortriazin-Schmelze (160°) werden durch eine Druckdüse in eine mit 215 Teilen Wasser von 3° gespeiste Dispergierkolonne unter Niveau eingesprüht. Die Dispergierkolonne wird von aussen gekühlt. so dass sich die Flüssigkeit nicht erwärmt. Man erhält eine 28%ige Suspension von Cyanurchlorid in Wasser, wobei die Korngrösse der Cyanurchlorid-Teilchen zwischen 0 und 20 µm liegt. Die Ausbeuteverluste infolge Hydrolyse liegen unter 0,1%.

## Ansprüche

1. Verfahren zur Herstellung einer Suspension von Cyanurchlorid in Wasser oder zur Umsetzung von Cyanurchlorid mit Ammoniak oder Aminen, indem man geschmolzenes Cyanurchlorid mit einer Temperatur zwischen 146 und 190°C in Wasser oder eine wässrige Ammoniak- oder Aminlösung oder -suspension mit einer Temperatur zwischen 0 und 25°C einsprüht, dadurch gekennzeichnet, dass man das geschmolzene Cyanurchlorid durch eine Düse, welche in die Flüssigkeit eintaucht, in Wasser oder eine wässrige Ammoniak- oder Aminlösung oder -suspension sprüht, wobei beim Starten des Einsprühvorgangs der Flüssigkeitspegel im Umgebungsbereich der Düse soweit abgesenkt wird bis die Düsenspitze nicht mehr in die Flüssigkeit eintaucht, und dass der Flüssigkeitspegel unmittelbar nach dem Starten wieder angehoben wird.

2. Verfahren gemäss Anspruch 1, dadurch gekennzeichnet, dass das geschmolzene Cyanurchlorid eine Temperatur zwischen 155 und 180°C aufweist.

3. Verfahren gemäss Anspruch 1, dadurch gekennzeichnet, dass man Ammoniak oder Amine als 5- bis 50%ige, vorzugsweise 10 bis 40%ige Lösung oder Suspension einsetzt.

4. Verfahren gemäss Anspruch 1, dadurch gekennzeichnet, dass die Düse geheizt wird.

5. Anlage zur Durchführung des Verfahrens nach Anspruch 1, mit einem Schmelzkessel und einem Tank für die Kühlflüssigkeit (Wasser bzw. wässrige Ammoniak- oder Aminlösung oder -suspension) und einer vom Schmelzkessel in den Kühlflüssigkeitstank führenden Schmelzeleitung,deren tankseitiges Ende eine Sprühvorrichtung aufweist, dadurch gekennzeichnet, dass in die Schmelzeleitung eine Druck- und Dosierpumpe eingebaut ist und zumindest diejenigen Teile der Schmelzeleitung, welche mit der Kühlflüssigkeit in Berührung kommen können, nach aussen wärmeisoliert und nach innen wirkend beheizbar sind, die Sprühvorrichtung durch eine Zerstäubungsdüse gebildet ist und unterhalb des Kühlflüssigkeitsniveaus innerhalb einer Tauchglocke angeordnet ist, welche über ein erstes Schliessorgan an eine Druckgasquelle angeschlossen und über ein zweites Schliessorgan entlüftbar ist, so dass bei Oeffnen des ersten Schliessorgans und geschlossenem zweiten Schliessorgan das Kühlflüssigkeitsniveau in der Glocke unter das Niveau der Sprühvorrichtung sinkt.

6. Anlage nach Anspruch 5, dadurch gekennzeichnet, dass die Schmelzeleitung als Kreislaufleitung ausgebildet ist, wobei nach der Druck- und Dosierpumpe ein Leitungszweig zur Düse und der andere zurück in den Schmelzkessel führt und in letzterem ein vorzugsweise einstellbares Druckhalteventil eingebaut ist.

7. Anlage nach Anspruch 5, dadurch gekennzeichnet, dass der Kühlflüssigkeitstank an einen äusseren Kreislauf mit einer Pumpe und einem Kühler angeschlossen ist.

8. Anlage nach Anspruch 7, dadurch gekennzeichnet, dass in den äusseren Kreislauf des Kühlflüssigkeitstanks vor der Kühlflüssigkeitspumpe eine Mühle zur Zerkleinerung von Agglomeraten eingebaut ist.

9. Anlage nach Anspruch 7 oder 8, dadurch gekennzeichnet, dass vom äusseren Kreislauf des Kühlilüssigkeitstanks nach der Mühle und vorzugsweise nach der Pumpe oder dem Kühler ein Suspensionsabzug-abgezweigt ist.

10. Anlage nach Anspruch 5, dadurch gekennzeichnet, dass die Düse in die nach aussen wärmeisolierten und nach innen beheizbaren Teile der Schmelzeleitung eingesteckt oder eingeschraubt ist.

11. Anlage nach Anspruch 10, dadurch gekennzeichnet, dass die wärmeisolierten Teile der Schmelzeleitung einen evakuierten Isolationsmantel aufweisen.

## Claims

1. A process for preparing a suspension of cyanuric chloride in water, or for reacting cyanuric chloride with ammonia or with amines, by spraying melted cyanuric chloride having a temperature between 146 and 190°C into water or an aqueous ammonia or amine solution or suspension having a temperature between 0 and 25°C, which process comprises spraying the melted cyanuric chloride through a nozzle, which is immersed in the liquid, into water or an aqueous ammonia or amine solution or suspension, in which, at the start of the spraying operation, the level of the liquid in the surrounding region of the nozzle is lowered until the nozzle tip is no longer immersed in the liquid, and in that the level of the liquid is raised again immediately after the start.

2. A process according to claim 1, wherein the melted cyanuric chloride has a temperature between 155 and 180°C.

3. A process according to claim 1, wherein ammonia or amines are used as a 5 to 50%, preferably 10 to 40%, solution or suspension.

4. A process according to claim 1, wherein the nozzle is heated.

5. An apparatus for performing the process according to claim 1, comprising a melting vessel and a tank for the cooling liquid (water or an aqueous ammonia or amine solution or suspension) and a melt line leading from the melt vessel into the cooling liquid tank, the end of the line on the tank side having a spray device,

wherein a pressure and metering pump is incorporated in the melt line, and at least those parts of the melt line which can come into contact with the cooling liquid are thermally insulated on the outside and are heatable on the inside, the spray device is formed by an atomising nozzle and is located below the level of the cooling liquid inside an immersion bell, which is connected by way of a first closure member, to a compressed gas source and from which air can be evacuated by way of a second closure member, so that upon opening of the first closure member and with the second closure member closed the level of cooling liquid in the bell sinks below the level of the spray device.

6. An apparatus according to claim 5, wherein the melt line is designed as a circulation line, one branch of the line, after the pressure and metering pump, leading to the nozzle and the other back into the melt vessel, and a preferably adjustable pressure-maintaining valve being incorporated in the latter branch.

7. An apparatus according to claim 5, wherein the cooling liquid tank is connected to an external circulation system with a pump and a cooler.

8. An apparatus according to claim 7, wherein there is incorporated in the external circulation system of the cooling liquid tank, before the cooling liquid pump, a mill for the comminution of agglomerates.

9. An apparatus according to either of claims 7 or 8, wherein, from the external circulation system of the cooling liquid tank, after the mill and preferably after the pump or the cooler, a suspension outlet branches off.

10. An apparatus according to claim 5, wherein the nozzle is inserted or screwed into the parts of the melt line which are thermally insulated on the outside and heatable on the inside.

11. An apparatus according to claim 10, wherein the thermally insulated parts of the melt line have an evacuated insulation jacket.

**Revendications**

1. Procédé pour la préparation d'une suspension de chlorure de cyanuryle dans l'eau ou pour la réaction du chlorure de cyanuryle avec de l'ammoniac ou des amines, dans lequel on pulvérise du chlorure de cyanuryle fondu, présentant une température comprise entre 146 et 190°C, dans de l'eau ou dans une solution ou suspension aqueuse d'ammoniac ou d'amine, présentant une température comprise entre 0 et 40°C, procédé caractérisé par le fait que l'on pulvérise le chlorure de cyanuryle fondu dans l'eau ou dans une solution ou suspension aqueuse d'ammoniac ou d'amine, au moyen d'une buse plongeant dans le liquide, le niveau du liquide autour de la buse étant abaissé, au début du processus de pulvérisation, jusqu'à ce que l'extrémité de la buse ne plonge plus dans le liquide, et que le niveau du liquide, immédiatement après ce début, est à nouveau relevé.

2. Procédé conforme à la revendication 1, caractérisé par le fait que le chlorure de cyanuryle fondu présente une température comprise entre 155 et 180°C.

3. Procédé conforme à la revendication 1, caractérisé par le fait que l'on utilise une suspension ou solution d'ammoniac ou d'amine à 5-50% et de préférence à 10-40%.

4. Procédé conforme à la revendication 1, caractérisé par le fait que l'on chauffe la buse.

5. Installation pour la réalisation du procédé conforme à la revendication 1, avec une chaudière de fusion et un réservoir pour le liquide froid (eau ou solution ou suspension aqueuse d'ammoniac ou d'amine) et une conduite pour la masse fondue, allant de la chaudière au réservoir et dont l'extrémité située du côté du réservoir présente un dispositif de pulvérisation, caractérisée par le fait que dans la conduite pour la masse fondue est incorporée une pompe de compression et de dosage, qu'au moins les parties de la conduite pour la masse fondue qui peuvent entrer en contact avec le liquide froid sont calorifugées extérieurement et peuvent être chauffées de façon que le chauffage s'exerce vers l'intérieur, et que le dispositif de pulvérisation est formé par une buse-pulvérisateur et disposé sous le niveau du liquide froid à l'intérieur d'une cloche de plongée qui est reliée à une source de gaz comprimé par un premier organe de fermeture et dont on peut chasser l'air par un deuxième organe de fermeture de façon à ce que, lorsqu'on ouvre le premier organe de fermeture alors que le deuxième est fermé, le niveau du liquide froid dans la cloche descende au-dessous du niveau du dispositif de pulvérisation.

6. Installation conforme à la revendication 5, caractérisée par le fait que la conduite pour la masse fondue constitue une conduite en circuit fermé, dont une branche part, après la pompe de compression et de dosage, en direction de la buse et dont l'autre retourne à la chaudière, et que sur cette dernière est incorporée une soupape de régulation de pression, de préférence réglable.

7. Installation conforme à la revendication 5, caractérisée par le fait que le réservoir de liquide froid est relié à un circuit extérieur fermé muni d'une pompe et d'un refroidisseur.

8. Installation conforme à la revendication 7, caractérisée par le fait qu'un moulin pour le broyage des agglomérats est incorporé dans le circuit extérieur du réservoir de liquide froid, avant la pompe à liquide froid.

9. Installation conforme à la revendication 7 ou 8, caractérisée par le fait qu'une sortie pour la suspension

est placée en dérivation sur le circuit extérieur du réservoir du liquide froid, après le moulin et de préférence après la pompe ou le refroidisseur.

10. Installation conforme à la revendication 5, caractérisée par le fait que la buse est enfoncée ou vissée dans les parties de la conduite pour la masse fondue qui sont calorifugées extérieurement et que l'on peut chauffer vers l'intérieur.

11. Installation conforme à la revendication 10, caractérisée par le fait que les parties calorifugées de la conduite pour la masse fondue présentent une chemise isolante où on fait le vide.

Fig. 1

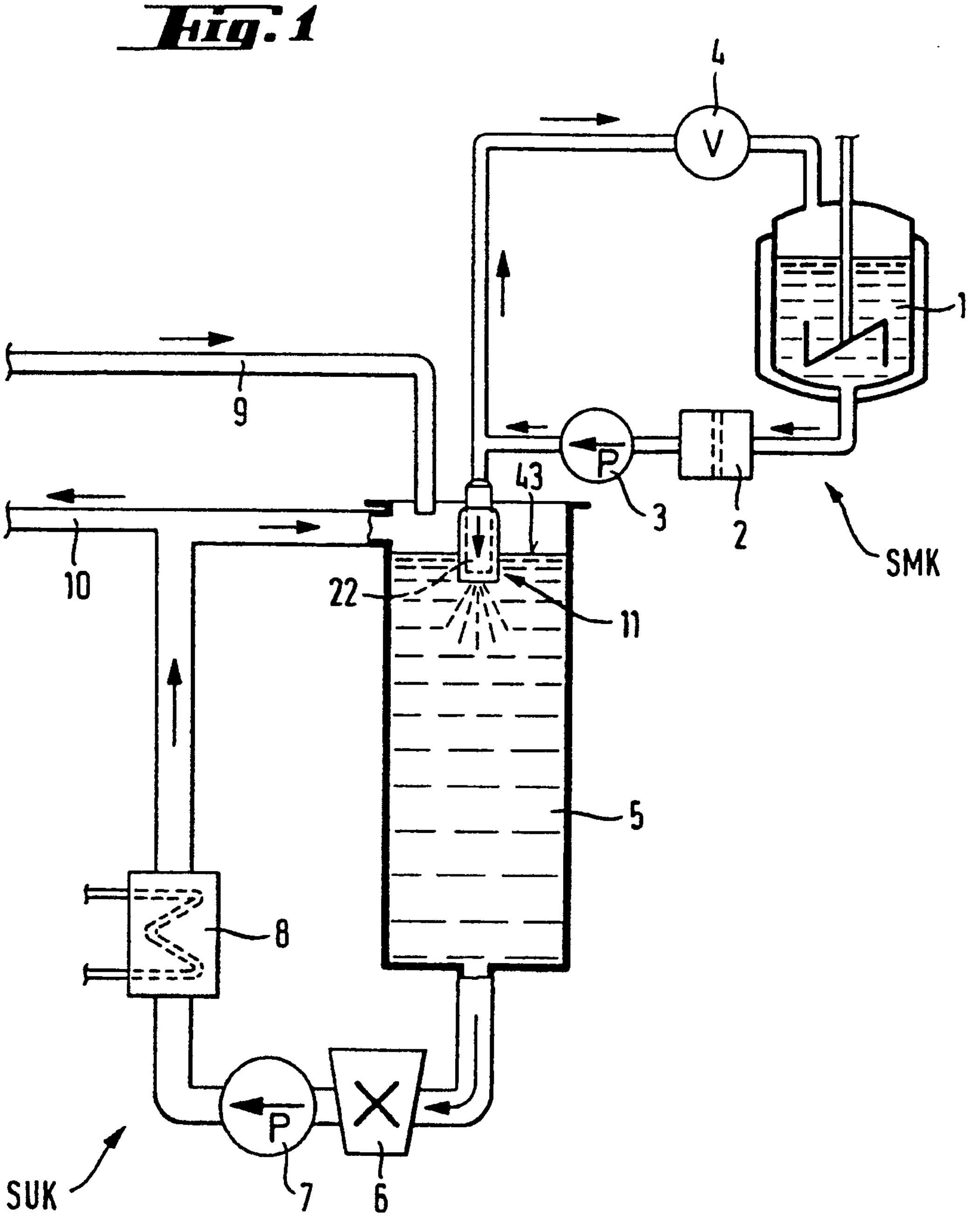

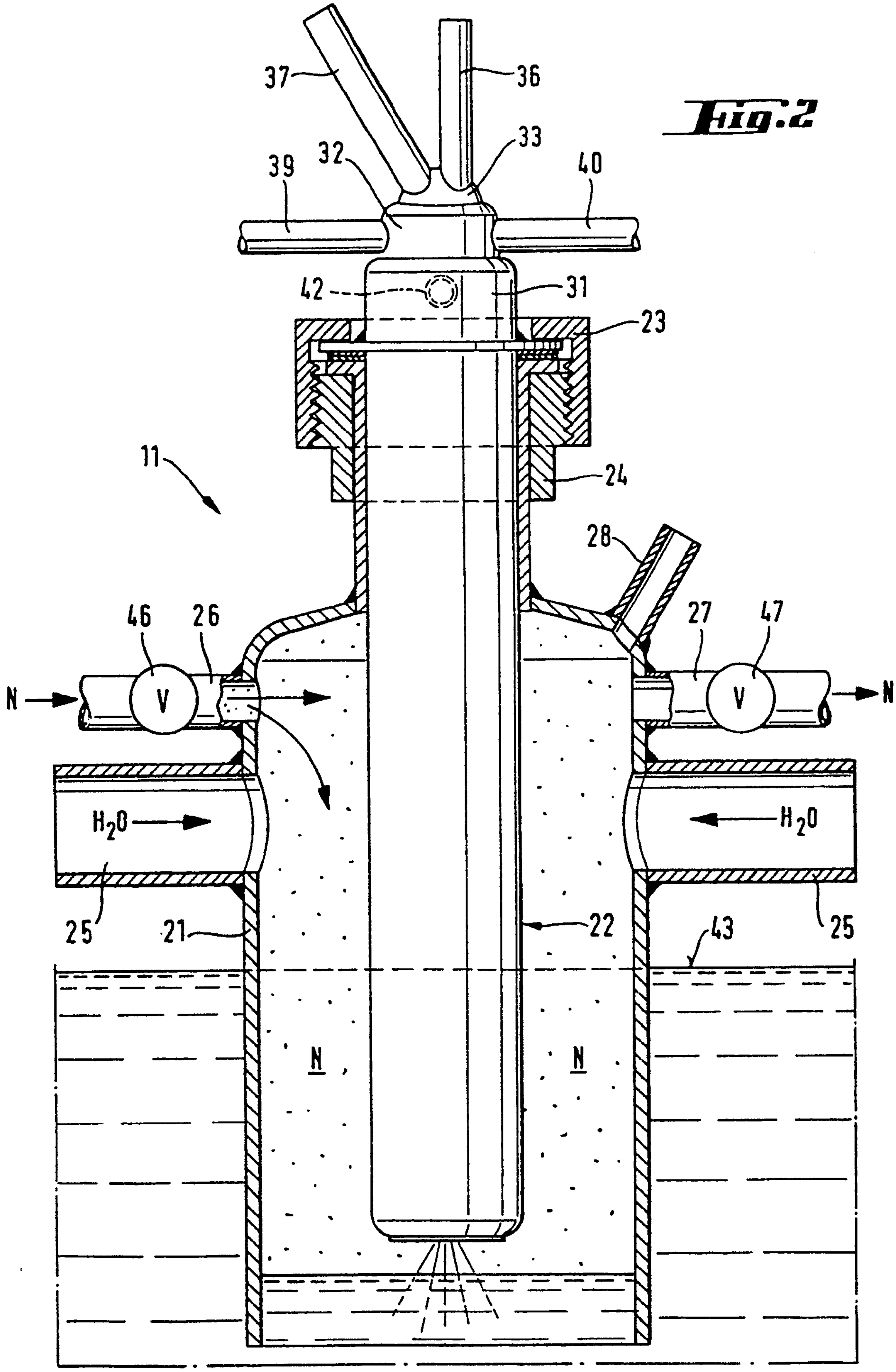
37
36
33
32
39
40
42
31
23
11
24
28
46
26
27
47
N
V
V
N
$H_2O$
$H_2O$
25
21
22
43
25
N
N

Fig.2

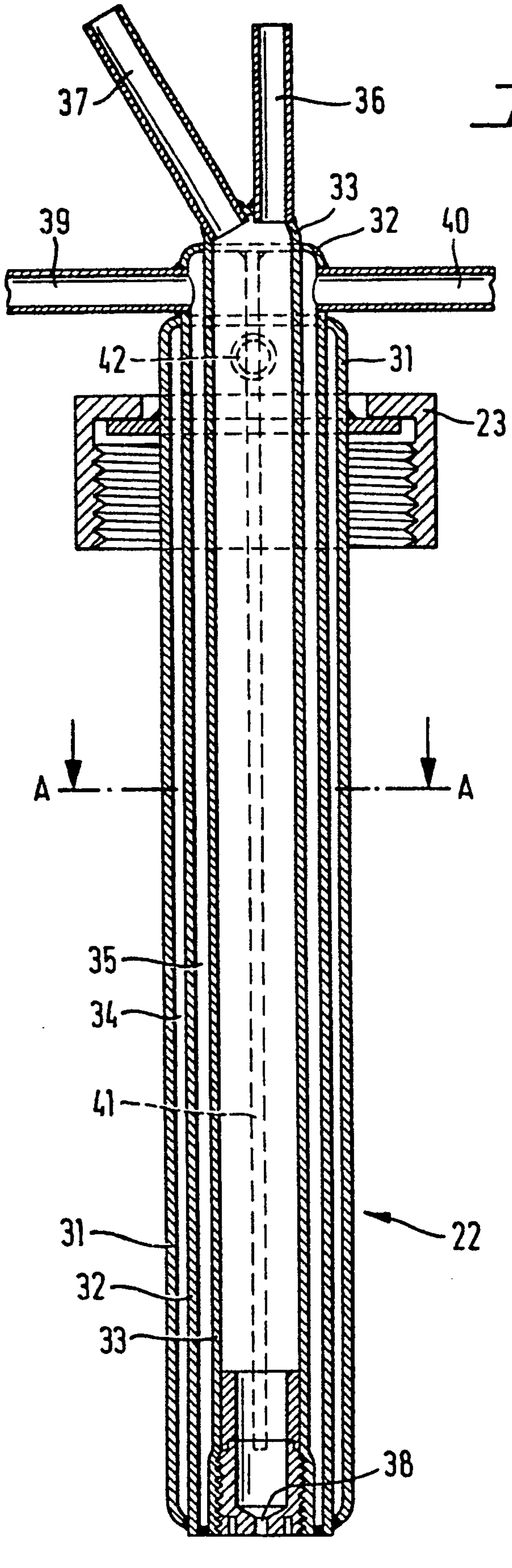
37
36
33
32
39
40
42
31
23
A
A
35
34
41
31
32
33
22
38

Fig. 3

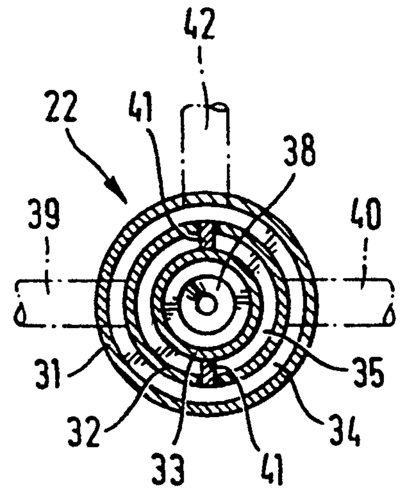
42
22
41
38
39
40
31
35
32
33
41
34

Fig. 4